# EUROPÄISCHE PATENTSCHRIFT

(11) **EP 2 882 366 B1**
(45) Veröffentlichungstag und Bekanntmachung des Hinweises auf die Patenterteilung: **04.01.2017**
(21) Anmeldenummer: 13745076.3
(22) Anmeldetag: 02.08.2013
(51) Int. Cl.: A61B 90/90, A61B 90/98

(54) **EINRICHTUNG UND VERFAHREN ZUM GLEICHZEITIGEN IDENTIFIZIEREN EINER VIELZAHL CHIRURGISCHER INSTRUMENTE**
DEVICE AND METHOD FOR SIMULTANEOUSLY IDENTIFYING A PLURALITY OF SURGICAL INSTRUMENTS
DISPOSITIF ET PROCÉDÉ POUR L'IDENTIFICATION SIMULTANÉE D'UN GRAND NOMBRE D'INSTRUMENTS CHIRURGICAUX

(30) Priorität: 08.08.2012 DE 102012107274
(43) Veröffentlichungstag der Anmeldung: 17.06.2015
(60) Teilanmeldung: 16195577.8
(73) Patentinhaber: Aesculap AG, 78532 Tuttlingen (DE)
(72) Erfinder: WEINERT, Markus, 78532 Tuttlingen (DE); GIORDANO, Nicola, 78056 Villingen-Schwenningen (DE); MORALES, Pedro, 78532 Tuttlingen (DE); WEISSHAUPT, Dieter, 78194 Immendingen (DE)
(74) Vertreter: Winter, Brandl, Fürniss, Hübner, Röss, Kaiser, Polte - Partnerschaft mbB
(86) Internationale Anmeldenummer: PCT/EP2013/066306
(87) Internationale Veröffentlichungsnummer: WO 2014/023663

(56) Entgegenhaltungen:
- EP-A1- 2 287 091
- WO-A2-2006/086603
- DE-A1-102005 047 522
- US-A1- 2004 118 920
- US-A1- 2006 043 177
- US-A1- 2007 210 159

## Beschreibung

Die Erfindung betrifft eine Einrichtung zum gleichzeitigen Identifizieren einer Vielzahl unterschiedlicher, chirurgischer Instrumente beziehungsweise Instrumentgruppen gemäß Anspruch 1 sowie ein Verfahren gemäß Anspruch 7.

### Technischer Hintergrund

Im Gesundheitswesen geht der Trend immer deutlicher in Richtung Einzelinstrumententracking, um auf Instrumenten- und Sterilgutebene die jeweiligen Medizinprodukte verfolgen zu können, um die Nutzungshäufigkeit, die Wartungsintervalle oder den aktuellen Lagerort ermitteln und verfolgen zu können. Zum Beispiel dienen Vollständigkeitskontrollen von Siebinhalten an unterschiedlichsten Stationen eines Instrumentenkreislaufs dazu, sicherzustellen, dass ein Sterilgut vollständig und in der richtigen Konfiguration zum Einsatz kommt.

Sie dienen ferner dazu, den großen und kostenintensiven Schwund an Instrumenten zu reduzieren.

Ferner dienen sie in besonderem Umfang der Patientensicherheit. Dieser Aspekt gewinnt vor dem Hintergrund an Bedeutung, dass bei einer Notfalloperation manchmal etwa 100 Druckrollen und 40 Kompressen neben den sonstigen Instrumenten zum Einsatz kommen. Ferner ist aus Krankenhausstatistiken bekannt, dass jährlich durchschnittlich 3000 Fälle auftreten, in denen Fremdköper wie Tücher, Tupfer oder ähnliches nach chirurgischen Eingriffen im Körper des Patienten verbleiben.

Neben traditionellen manuellen Methoden wie Einsortieren und Zählen kommen mittlerweile verschiedenste Technologien zur Anwendung, welche die Erfassung von chirurgischen Instrumenten unterstützen oder sogar vollautomatisiert ablaufen lassen. Dazu zählen beispielsweise Funkerkennung mittels einer RFID (radio-frequency identification) - Technologie, Gewichtsmessung, optische Erkennung, Barcodeerkennung, etc.

Bisherige Lösungen basieren oftmals auf dem einzelnen Auslesen von Instrumenten beziehungsweise der Sterilgüter, was zeitintensiv ist. Daher wurde das einzelne, händische Erfassen von Einzelinstrumenten zur automatisierten Einzelinstrumentenerfassung weiterentwickelt. Dabei steht vor allem die Verkürzung der Durchlaufzeiten im Vordergrund, um Zeit sowie Kosten zu sparen. Ebenso ist eine hohe Prozesssicherheit zwingend erforderlich, damit eine Automatisierung zum gleichzeitigen Einsparen von Zeit und Kosten ermöglicht wird. An der Einzelinstrumentenerfassung ist nachteilig, dass der Leseprozess bei einer Vielzahl von Gegenständen beziehungsweise Instrumenten viel Zeit in Anspruch nimmt.

Eine weitere bekannte Lösung ist daher, eine Vielzahl von Instrumenten gleichzeitig zu erfassen, um auf diese Weise die Durchlaufzeiten insbesondere im Gegensatz zu Einzellesungen beim Instrumententracking (z.B. bei Data Matrix) zu reduzieren. Wie in US 7,118,029 B2 vorgeschlagen, werden dabei mehrere mit RFID-Tags markierte Instrumente (RFID-Instrumente), die in einem Sieb liegen, gleichzeitig erfasst, mit Sieblisten verglichen und gegebenenfalls falsch einsortierte Instrumente erkannt.

Da der RFID-Transponder am chirurgischen Instrument nicht beliebig groß gestaltet werden kann, muss in der Regel auf passive RFID-Tags zurückgegriffen werden, die durch das vom RFID-Lesegerät erzeugte hochfrequente elektromagnetische Wechselfeld betrieben werden. Da das Strahlungsfeld des Strahlungsfeldes quadratisch mit der Entfernung abnimmt, muss mit einer recht hohen Sendeleistung gearbeitet werden. Gerade im medizinischen Umfeld ist dies jedoch nur begrenzt möglich, um etwaige Interferenzen mit anderen hochempfindlichen Geräten, insbesondere im OP-Saal, zu vermeiden.

Bei dieser sog. Pulkerfassung können Überlappungen und Verdeckungen im Sieb oder Behälter dazu führen, dass einige der zu erfassenden Instrumente von dem verwendeten Lesegerät falsch oder gar nicht erfasst und somit übersehen werden. Ferner führen die damit verbundene schlechte Erkennungswahrscheinlichkeit sowie die geringere Prozesssicherheit dazu, dass der Lesevorgang nicht automatisiert werden kann. Folglich sind auch bei dieser Lösung erhöhte Durchlaufzeiten und Kosten in Kauf zu nehmen.

US 2007/0210159 A1 offenbart eine Vorrichtung für eine Identifizierung einzelner chirurgischer Instrumente anhand eines auf dem Instrument aufgebrachten RFID-Labels und einer Markierung.

US 2006/0043177 A1 offenbart eine Vorrichtung sowie ein Verfahren für eine automatische Erfassung eines chirurgischen Instrumentenfachs, an dem ein RFID-Label aufgebracht ist, sowie dessen Inhalt an chirurgischen Instrumenten, welche ebenfalls mit RFID-Label etikettiert sind.

Da mit den bereits bekannten Systemen entweder eine hohe Prozesssicherheit auf Kosten der Durchlaufgeschwindigkeit oder eine hohe Durchlaufgeschwindigkeit auf Kosten der Prozesssicherheit und damit Automatisierbarkeit realisiert werden, besteht Handlungsbedarf in der Weiterbildung der Lesesysteme.

Vor diesem Hintergrund liegt der Erfindung die Aufgabe zu Grunde, eine Einrichtung sowie ein Verfahren bereitzustellen, die zuverlässig eine Vielzahl von unterschiedlichen Instrumenten gleichzeitig und mit einer hohen Erkennungswahrscheinlichkeit erfassen können.

Diese Aufgabe wird hinsichtlich der Einrichtung mit den Merkmalen des Patentanspruchs 1 und hinsichtlich des Verfahrens mit den Merkmalen des Patentanspruchs 7 gelöst.

Vorteilhafte Weiterbildungen der Einrichtung sind in den Patentansprüchen 2 bis 6 beschrieben.

In der erfindungsgemäßen Einrichtung kommt eine Kombination aus einer ersten Erfassungseinrichtung mit einer ersten Erfassungstechnologie zum Erfassen von Instrumenten und Instrumenteninformationen, insbesondere einer RFID-Erkennung, und einer zweiten Erfassungseinrichtung mit einer zweiten, von der ersten verschiedenen Erfassungstechnologie zum Erfassen von Instrumenten und Instrumenteninformationen sowie eine Abgleicheinrichtung zum Abgleichen der Erfassungsergebnisse der beiden Erfassungstechnologien zum Einsatz. Stimmen die abgeglichenen Erfassungsergebnisse überein, gibt die Abgleicheinrichtung hinsichtlich der Richtigkeit der erfassten Instrumente eine positive Beurteilung aus bzw. verifiziert die Erfassungsergebnisse, so dass entsprechend weitere Schritte ergriffen werden können, z.B. dass die erfassten Instrumente registriert oder dass ein Bediener darüber informiert wird, dass alle Instrumente richtig erfasst worden sind.

Die Einrichtung ermöglicht somit die Pulkerfassung einer Vielzahl von unterschiedlichen Instrumenten bzw. Sterilguts. Sie hat den Vorteil, dass in logistischen Prozessen wie beispielsweise bei der automatischen Instrumentenerkennung die in einem Siebkorb oder Container liegenden Instrumente ohne Kosten verursachende Vereinzelung gleichzeitig beziehungsweise in einem Lesevorgang erfasst werden.

Ferner werden durch die schnelle Pulkerfassung an unterschiedlichsten Stellen im Instrumentenkreislauf beziehungsweise Prozess des Instrumentariums ohne größeren zeitlichen Aufwand bislang nicht verfügbare Prozessinformationen, wie beispielsweise Verweilzeiten vor und / oder nach den jeweiligen Prozessstationen, gewonnen. Mit Hilfe dieser auswertbaren Informationen kann sowohl die Prozesssicherheit als auch der Prozessablauf verbessert werden.

Darüber hinaus wird durch den Einsatz von zumindest zwei unterschiedlichen Erfassungstechnologien sichergestellt, dass alle zu erfassenden Instrumente erfasst werden und etwaige nicht erfassbare Bereiche beziehungsweise Instrumente der einen Technologie von der anderen erfasst werden oder dass zumindest die Erkennungswahrscheinlichkeit und damit die Prozesssicherheit wesentlich erhöht werden kann. Zudem werden mit der zweiten Erfassungstechnologie Instrumente erfasst, welche generell, z.B. bei einem verlorenen gegangen RFID-Tag oder Barcode, oder aufgrund der momentanen Unterschiede für die erste Erfassungstechnologie nicht erkennbar sind.

Demnach werden durch die Kombination der jeweiligen Technologievorteile insbesondere die Vorteile der verschiedenen Erfassungstechnologien ausgenutzt. Auf diese Weise werden Kombigeräte mit mehreren integrierten Instrumentenerfassungssystemen mit dem Ziel geschaffen, die Erkennungswahrscheinlichkeit von Siebkorbinhalten zu maximieren. So ermöglicht die redundante Erfassung der Instrumente durch zwei unterschiedliche Erfassungstechnologien erstmals, dass ein Erfassungsergebnis als ein falsches Ergebnis erkannt und dem Bediener mitgeteilt wird.

Zudem werden die benötigten Lesevorgänge, bis ein richtiges Erfassungsergebnis vorliegt, reduziert, so dass durch die Reduzierung der Fehlerausgabe die Durchlaufgeschwindigkeiten erhöht werden, wodurch sich die Prozesszeiten deutlich reduzieren. Das ermöglicht wiederum die Automatisierung der Instrumentenerkennung.

Im Bereich der Medizintechnik, insbesondere in der Chirurgie herrschen vergleichsweise strenge Anforderungen für den Operationssaal. Da hier wie eingangs bereits erwähnt empfindliche technisch Geräte zum Einsatz kommen, dürfen diese auf keinen Fall durch die Funkleistung der RFID-Einrichtung gestört werden. Infolge der Reduzierung der Leseenergie verringert sich auch die Erkennungswahrscheinlichkeit dieser Erfassungstechnologie. Daher ist es gerade in solch einem Fall von besonderem Vorteil, dass die RFID-Technologie durch eine weitere, die im Operationssaal befindlichen Geräte nicht oder weniger beeinflussende Technologie unterstützt wird.

Bei der vorliegenden Erfindung ist vorteilhaft, dass die Erfassungsergebnisse der beiden Technologien durch die Abgleicheinrichtung abgeglichen werden. Damit wird sichergestellt, dass alle zu erfassenden Instrumente richtig erfasst und identifiziert wurden und ein fehlerhaftes Erfassungsergebnis erkannt wird. Beim Feststellen einer Abweichung zwischen den beiden Ergebnissen können weitere Maßnahmen wie beispielsweise ein erneuter Lesevorgang, eine Veränderung der Menge oder Lage der zu erfassenden Instrumente eingeleitet werden.

Jede Erfassungstechnologie erfasst unter Umständen mehr oder weniger Informationen zu einem bestimmten erfassten Instrument. Nach Verifikation der Erfassungsergebnisse kann das Erfassungsergebnis derjenigen Erfassungseinrichtung weiterverwendet oder -verarbeitet werden, welches die meisten Informationen zu den erkannten Instrumenten liefert. Gegebenenfalls können so jedoch auch die von einer Erfassungseinrichtung zu einem bestimmten Instrument erfassten Informationen um die von der anderen Erfassungseinrichtung ermittelten Informationen ergänzt werden.

Durch die Verwendung von zwei unterschiedlichen Technologien und den Vergleich der beiden Erfassungsergebnisse miteinander kann die erfindungsgemäße Einrichtung somit autark, d.h. ohne irgendwelche Vergleiche mit vorgegebenen Inhaltslisten, arbeiten, da sie gewissermaßen selbst einen Plausibilitätstest durchführt.

Dennoch kann in einer Weiterbildung die Abgleicheinrichtung die beiden Erfassungsergebnisse mit einem vorbestimmten Datensatz, insbesondere einer Instrumenten- oder einer Siebkorbliste, in welcher der gesamte Siebkorbinhalt aufgeführt ist, vergleichen, wobei die Abgleicheinrichtung eine positive Beurteilung ausgeben kann, wenn zumindest eines der beiden Erfassungsergebnisse mit dem vorbestimmten Datensatz übereinstimmt.

Der vorbestimmte Datensatz kann eine extern erzeugte und der Einrichtung eingegebene Liste beziehungsweise Instrumentenliste sein, welche die einzelnen Instrumente von vorbestimmten Instrumentenkonfigurationen beziehungsweise Sets beinhaltet. Ebenso kann es sich bei der Liste um eine in der Einrichtung intern erzeugte Liste handeln, die basierend auf vorangehende Lesevorgänge erzeugt und zwischengespeichert wurde.

Dies ist insbesondere für Vollständigkeitskontrollen vor, während und nach Packvorgängen von Siebkörben von Bedeutung.

Insbesondere bei Instrumentenmanagementsystemen sind Vollständigkeitskontrollen von besonderer Bedeutung, da diese ständig gepflegt und auf den aktuellen Stand gebracht werden müssen.

Wenn zumindest eine der Erfassungseinrichtungen alle auf der Instrumentenliste aufgeführten Instrumente erkannt hat, kann davon ausgegangen werden, dass die Erfassung dieser Einrichtung richtig ist. Wird also nach Abgleichen des ersten oder zweiten Erfassungsergebnisses mit dem hinterlegten Datensatz festgestellt, dass zumindest ein Erfassungsergebnis alle auf der Liste aufgeführten Instrumente erfasst hat, entfallen somit alle weiteren Lesevorgänge, was wiederum in einer weiteren Zeitersparnis resultiert.

Des Weiteren hat der Datenlistenabgleich bei einer Mehrfacherkennung den Vorteil, dass Vollständigkeitskontrollen beim Zusammenstellen von Siebkörben und Leihsieben durchgeführt werden können. Ebenso können Vollständigkeitskontrollen beispielsweise für Siebkörbe mit einer bestimmten anwendungsspezifischen Konfiguration vorgenommen werden.

Insbesondere ist die Gegenkontrolle mit hinterlegten Listen dahingehend vorteilhaft, als dass ein unerwünschter Verlust teilweise kostenintensiver Instrumente und / oder Sterilguts minimiert wird. Das wird dadurch erreicht, dass die Instrumente vor und nach jeder Art von Gebrauch und / oder Waschvorgang und ähnliches erfasst werden.

Ganz besonders vorteilhaft ist die höhere Patientensicherheit durch Vollständigkeitskontrollen der verwendeten Instrumente vor und nach der Operation. Dadurch werden einerseits verwendete und noch fehlende Instrumente identifiziert. Ferner wird auf diese Weise ebenso festgestellt, dass noch Instrumente vorliegen, welche gegebenenfalls hätten verwendet werden sollen. Dieser Aspekt ist insbesondere vor dem Hintergrund, dass Operationen erst bei Vollzähligkeit aller verwendeten Instrumente abgeschlossen werden dürfen, und jede OP-Minute teuer ist, gerade bei Verwendung vieler Instrumente von sehr großer Bedeutung.

In einer Weiterbildung kann die Einrichtung zum gleichzeitigen Identifizieren von Instrumenten derart ausgebildet sein, dass die Abgleicheinrichtung eine positive Beurteilung ausgibt, wenn zumindest eine Kombination der Erfassungsergebnisse mit einem vorbestimmten Datensatz übereinstimmt.

Das ist besonders vorteilhaft, wenn manche zu erfassenden Instrumente beispielsweise wegen einer ungünstigen Positionierung von der einen Erfassungstechnologie nicht erfasst werden können, während andere Instrumente wiederum von der anderen Erfassungstechnologie nicht erfasst werden können. Durch diese Weiterbildung werden alle erfassten Instrumente jeweils mit einem Datensatz verglichen. Wenn dann die jeweils von den beiden Erfassungstechnologien erfassten Instrumente zumindest in der Zusammenschau dem vorbestimmten Datensatz entsprechen, gibt die Abgleichseinrichtung eine positive Beurteilung aus.

Besonders bevorzugt ist, dass die Einrichtung zeitlich und / oder örtlich vor- oder nachgeschaltet eine Rütteleinrichtung und / oder eine Fließbandeinrichtung zum Vereinzeln der Instrumente, und eine Steuerungsvorrichtung zum Steuern der Rüttel- und / oder Fließbandeinrichtung aufweisen kann. Diese betätigt zumindest einmal die Rüttel- und / oder Fließbandeinrichtung für eine vorbestimmte Zeitdauer, und bevor die Instrumenteninformationen erneut von der Abgleicheinrichtung erfasst und abgeglichen werden.

Bevor das Lesen der Instrumente beginnt, kann demnach die Rüttel- und / oder Fließbandeinrichtung solange betätigt werden, bis die zu erfassenden Instrumente in dem Siebkorb weitgehend verteilt sind. Diese Dauer kann entweder eine vorbestimmte oder von dem Bediener überwachte Dauer sein. Während beziehungsweise nach einem Lesevorgang kann die Einrichtung ebenfalls betätigt werden, um etwa die aktuelle Instrumentenordnung zu verändern.

Üblicherweise schließt sich einem Rüttelvorgang ein erneuter Lesevorgang an, wobei dieser Ablauf beliebig oft, vorzugsweise jeweils einige Male wiederholt werden kann, um schließlich alle Instrumente zu erfassen.

Vorteilhaft dabei ist, dass gegenseitige Überlappungen und / oder Abdeckungen von Instrumenten sowie ungünstige Lagen der zu erfassenden Instrumente beseitigt werden. Dies erzielt den Vorteil, dass eine höhere Erkennungswahrscheinlichkeit und damit Prozesssicherheit erreicht werden. Ferner wird eine noch bessere Automatisierung des Lesevorgangs einer der Mehrfacherkennung einer Vielzahl von Instrumenten erreicht, in dem die Einrichtung zum Identifizieren von Instrumenten im Fall einer negativen Beurteilung eine eigene Abhilfemaßnahme, nämlich das Betätigen der Rüttel- und/oder Fließbandeinrichtung zum Beabstanden der Instrumente voneinander, aufweist.

In einer Weiterbildung der Erfindung kann sie eine Anzeigeeinrichtung aufweisen, mit der Beurteilungen über Vollständigkeit und Richtigkeit der Erfassungsergebnisse sowie die erfassten Instrumente zur Anzeige gebracht werden. Ferner kann bei unvollständiger Erfassung angezeigt beziehungsweise hervorgehoben werden, welche Instrumente der aktuellen Instrumentenliste noch nicht erfasst wurden. Demnach kann der Bediener gegebenenfalls gezielt nach diesen Instrumenten suchen, diese händisch nachtragen und auf diese Weise den Lesevorgang erfolgreich abschließen.

Mit der Anzeige können zudem vom Datensatz enthaltene produktspezifische Zusatzinformationen wie beispielsweise eine spezielle Behandlung und / oder Handhabung ausgewählter Instrumente angezeigt werden. Ferner kann die Zusatzinformation darin bestehen, dass einem erfassten Instrument bestimmte weitere Instrumente beizulegen sind oder ein Bauteil davon wie beispielsweise ein Verschleißteil auszutauschen ist.

Vorteilhafterweise wird so direkt an der Anzeigeeinrichtung angezeigt, dass beispielsweise eine Inspektion ansteht, es sich um eine Leihgabe handelt oder ein Instrument zu entsorgen ist.

Handelt es sich bei den erfassten Instrumenten um zu reinigende Instrumente, die aus einem Operationssaal stammen, und ist eine Demontage dazu erforderlich, werden außerdem hilfreiche Informationen wie Demontageanleitungen beziehungsweise -bilder angezeigt. Wenn ausnahmsweise ein spezielles Waschprogramm oder ein Reinigungszusatz beizugeben ist, wird der Bediener auch hierauf hingewiesen.

Ganz besonders vorteilhaft erweist es sich, wenn die Einrichtung ferner mit Mittel zum Ändern einer relativen Orientierung und / oder eines relativen Abstands zwischen den Instrumenten und den Erfassungseinrichtungen versehen ist.

Vorteilhafterweise können in einem Fall, wenn noch keine positive Beurteilung ausgegeben werden kann, sowohl eine Orientierung als auch eine Distanz zwischen den Instrumenten und den Erfassungseinrichtungen durch Bewegen von zumindest einer Erfassungseinrichtung und / oder der Instrumente variiert werden, um so die Wahrscheinlichkeit einer kompletten Auslesung zu erhöhen. Dazu ist eine Erfassungseinrichtung relativ bewegbar zur Instrumentenunterlage beziehungsweise - behälter und umgekehrt.

Folglich werden auch von der Erfassungseinrichtung weiter entfernte beziehungsweise verdeckte Instrumente erfasst, ohne die Leseenergie erhöhen zu müssen. Dies ist insbesondere dann von Vorteil, wenn die Leseenergie die Umgebung möglichst wenig beeinflussen soll. Auf diese Weise werden die Instrumente vollständig erfasst, ohne das Gehäuse, in dem die Erfassungseinrichtung installiert ist, abschirmen oder stärker abschirmen zu müssen.

Um die Erfassungsergebnisse einer Plausibilitätskontrolle unterziehen zu können, kann die Einrichtung der vorliegenden Erfindung ferner eine Waage zur Gewichtserfassung aufweisen. Die Abgleicheinrichtung nimmt in Abhängigkeit der identifizierten Instrumente und ihrer im Datensatz hinterlegten Gewichtsangaben eine Plausibilitätskontrolle der Auswertung vor, indem sie ein erfasstes Ist-Gewicht mit einem hinterlegten Soll-Gewicht vergleicht. Basierend auf der Plausibilitätskontrolle gibt sie eine negative Beurteilung aus, wenn das Ist-Gewicht nicht mit dem Soll-Gewicht übereinstimmt, und gibt eine positive Beurteilung aus, wenn das Ist-Gewicht mit dem Soll-Gewicht übereinstimmt.

Mit einer Gewichtserfassung ist eine Automatisierung besser realisierbar, da Fehlerausgaben durch eine weitere Kontrolle der Erfassungsergebnisse reduziert werden.

In einer besonders bevorzugten Weiterbildung kann die Einrichtung als ein sog. Tunnelreader ausgebildet sein, der ein abgeschirmtes oder abschirmbares Gehäuse aufweist, in dem beziehungsweise innerhalb dessen zumindest zwei, vorzugsweise alle unterschiedlichen Erfassungseinrichtungen angeordnet sind und eine integrierte Einheit bilden. Somit können die zu erfassenden Instrumente auf einmal von dem Tunnelreader erfasst werden. Ferner kann der Tunnelreader beim Lesevorgang durch Schließen einer oder mehreren Klappen vollständig von der Umgebung abgeschirmt werden, um einen Außenbereich, insbesondere den OP-Bereich, durch den Lesevorgangs nicht zu beeinträchtigen. Das hat den Vorteil, dass die Leseenergien höher sein können und folglich die Erkennungswahrscheinlichkeit verbessert wird.

Dabei kann ein Einschubfach sowohl manuell, als auch automatisch durch eine Klappe betätigbar sein.

Alternativ kann der Tunnelreader insbesondere im OP-Bereich in Form einer Durchreiche mit offener Rückwand ausgebildet sein. Auf diese Weise wird das Sterilgut direkt erfasst und in den OP-Bereich gereicht. Die im OP-Bereich verwendeten Instrumente können dann auf dem gleichen Weg zurück gegeben werden. Diese Weiterbildung hat den Vorteil, dass unmittelbar nach Gebrauch der Instrumente eine Vollständigkeitskontrolle durchgeführt wird, wodurch die Patientensicherheit deutlich verbessert wird. Das ist insbesondere von Vorteil, da während der Operation kein zusätzliches Personal benötigt wird, das sich um das Zählen der Instrumente kümmern muss.

Besonders vorteilhaft kann der Tunnelreader ausgestaltet sein, wenn die Durchreiche nur einseitig, vorzugsweise auf der Seite des OP-Bereichs eine verschließbare Klappe aufweist, damit ein Einschubfach nach dem Einbringen des zu lesenden Siebs geschlossen werden kann. Der Innenraum des Tunnelreaders kann dann vordem Lesevorgang vom OP-Bereich im Wesentlichen vollständig abgeschirmt werden, so dass dieser auch bei höheren Wattzahlen der RFID-Erkennung keine Beeinträchtigung erfährt.

Somit ist es besonders vorteilhaft, wenn der Tunnelreader im Zuge einer Operation, insbesondere vor dem Hintergrund der Patientensicherheit, zur Anwendung kommt. Die Anwendung ist allerdings nicht hierauf beschränkt, sondern kann ebenso vorzugsweise vor und nach einem Transport von Instrumenten, einem Reinigungs-/Desinfektionsvorgang oder Sterilisationsvorgang zum Einsatz kommen. Dies trifft gleichermaßen auf Packvorgänge und wie Entnahmen zur Reparatur einzelner Instrumente zu.

In einem erfindungsgemäßen Verfahren zum gleichzeitigen Identifizieren von einer Vielzahl, vorzugsweise unterschiedlicher, chirurgischer Instrumente beziehungsweise Instrumentgruppen werden zunächst durch eine erste und eine zweite Erfassungseinrichtung jeweils die Instrumente erfasst und ein erstes und ein zweites Erfassungsergebnis ausgegeben, welche anschließend durch eine Abgleicheinrichtung miteinander verglichen werden, wobei eine positive Beurteilung dieses Vergleichs ausgegeben wird, wenn die beiden Erfassungsergebnisse übereinstimmen.

Vorteilhafterweise werden die Erfassungsergebnisse in einem weiteren Schritt mit einer ersten Instrumentenliste verglichen. Wenn zumindest eines der Erfassungsergebnisse mit der Instrumentenliste übereinstimmt, wird eine positive Beurteilung ausgegeben.

Somit kann auch dann eine positive Beurteilung bestimmt werden, wenn zumindest eines der Erfassungsergebnisse mit der Instrumentenliste übereinstimmt.

Besonders vorteilhaft ist der Verfahrensschritt, in dem eine Kombination der Erfassungsergebnisse mit der vorbestimmten Instrumentenliste verglichen wird. Dieser Vergleich führt zu einer positiven Bestimmung, wenn zumindest eine Kombination der Erfassungsergebnisse mit der Instrumentenliste übereinstimmt.

Folglich kann eine positive Beurteilung bestimmt werden, wenn keines der Erfassungsergebnisse, aber zumindest eine Kombination von ihnen mit der Instrumentenliste übereinstimmt.

Besonders vorteilhaft erweist sich das oben genannte Verfahren in Verbindung mit einer Rüttelvorrichtung, welche für eine vorbestimmte Zeitdauer betätigt werden kann. Dieser Schritt ist insbesondere dann von Bedeutung, wenn in einem ersten Erfassungsschritt keine positive Beurteilung bestimmt wurde. Im Anschluss der Betätigung der Rüttelvorrichtung werden die Erfassungsergebnisse erneut erfasst.

Je nach Bedarf können Dauer, Art sowie Wiederholungen der Rüttelvorrichtungsbetätigung automatisch nach einem vorbestimmten Schema oder durch einen Bediener gesteuert werden. Damit ist eine Anpassung an den Siebkorbinhalt möglich.

Das Verfahren realisiert somit ebenfalls die eingangs im Zusammenhang mit der erfindungsgemäßen Einrichtung genannten Vorteile.

### Kurze Beschreibung der Zeichnungen

Im Folgenden wird ein Ausführungsbeispiel einer erfindungsgemäßen Einrichtung zum gleichzeitigen Identifizieren von einer Vielzahl unterschiedlicher, chirurgischer Instrumente unter Bezugnahme auf die beigefügten Zeichnungen näher erläutert.

Es zeigen:
Figur 1 ein schematische Schnittdarstellung eines Tunnelreaders der vorliegenden Erfindung in einer Seitenansicht; und
Figur 2 ein Ablaufdiagramm zur Erläuterung eines gleichzeitigen Identifizierens einer Vielzahl von chirurgischen Instrumenten oder Instrumentgruppen.

### Detaillierte Beschreibung der Zeichnungen

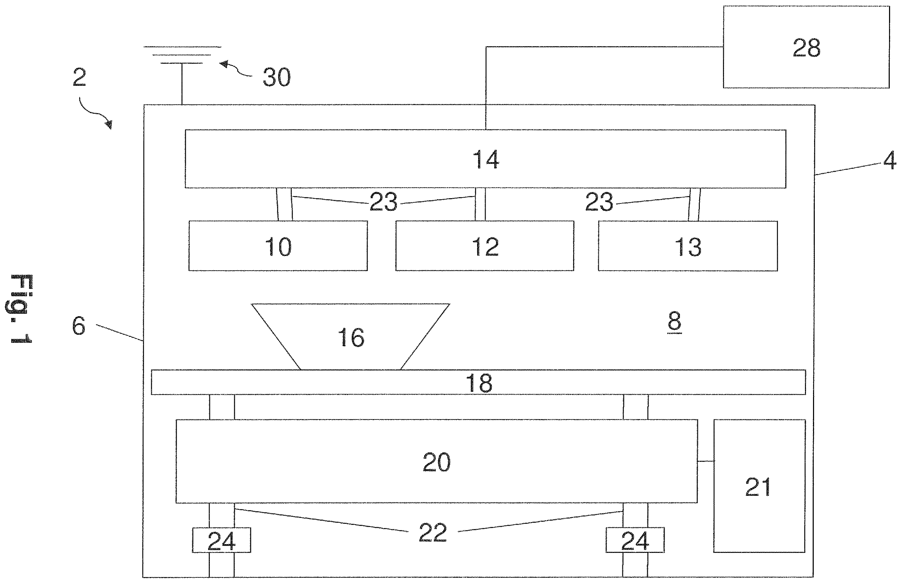

Fig. 1 zeigt beispielhaft in einer Seitenansicht einen Tunnelreader 2 zum gleichzeitigen Identifizieren von einer Vielzahl an vorzugsweise unterschiedlicher, chirurgischer Instrumente. Dessen Gehäuse 4 hat im Wesentlichen die Form eines Quaders, der in Längsrichtung des Tunnelreaders 2 an seinen beiden Seitenflächen jeweils eine Öffnung 6 aufweist, welche die beiden Enden eines längs durch das Gehäuse 4 verlaufenden Durchgangs 8 mit vorzugsweise rechteckigem Querschnitt bilden. Die Öffnungen 6 können jeweils mit einer (nicht gezeigten) Klappe geschlossen werden.

Der Tunnelreader 2 weist eine RFID-Leseeinrichtung beziehungsweise RFID-Reader 10 als eine erste Erfassungseinrichtung, eine optische Leseeinrichtung bzw. Kamera 12 als zweite Erfassungseinrichtung, einen Barcode-Scanner 13 als dritte sowie eine Waage 24 als vierte Erfassungseinrichtung auf, welche alle mit einer Abgleicheinrichtung 14 vernetzt sind und in Datenaustausch stehen. Die Einrichtungen 10, 12, 13, 14, 24 sind innerhalb des Gehäuses 4 angeordnet. Die erste bis dritte Erfassungseinrichtung 10, 12, 13 sind an einem oberen Abschnitt des Durchgangs 8 so angeordnet, dass sie einem in den Tunnelreader 2 eingeführten Behältnis beziehungsweise Siebkorb 16 gegenüber stehen, während sich die Waage 24 zur Gewichtsmessung unterhalb des Siebkorbs 16 befindet.

Der Durchgang 8 weist einen Boden zum Tragen des Siebkorbs 16 auf, wobei dieser vorzugsweise als ein Fließband 18 ausgebildet ist. Das Fließband 18 ist nicht direkt, sondern über eine Rütteleinrichtung 20, mit dem Gehäuse 4 verbunden, wobei die Rütteleinrichtung 20 vorzugsweise unterhalb des Fließbands 18 oder an dessen Seitenabschnitten angebracht, mit diesem gekoppelt und ferner mit dem Gehäuse 4 verbunden ist und es in Vibrationen versetzt. Ferner weist die Rütteleinrichtung 20 eine Steuerungsvorrichtung 21 zum Steuern der Rütteleinrichtung 20 und des Fließbands 18 auf, welche mit der Abgleicheinrichtung 14 kommuniziert. In Abhängigkeit einer Beurteilung der Abgleicheinrichtung 14 steuert die Steuerungsvorrichtung 21 das Fließband 18 und / oder die Rütteleinrichtung 20. Auf diese Weise kann sowohl die Orientierung der Instrumente im Siebkorb 16, als auch die Position des gesamten Siebkorbs 16 zu den Erfassungseinrichtungen 10, 12, 13 verändert werden. Zusätzlich kann das Fließband 18 durch kurze und schnell aufeinander folgende Richtungswechsel des Fließbands 18 den Siebkorb 16 schütteln.

Des Weiteren sind die Erfassungseinrichtungen 10, 12, 13 jeweils über Teleskophaltevorrichtungen 23 mit Elektromotor (nicht dargestellt) am Gehäuse 4 so befestigt, dass sie einzeln oder zusammen heruntergelassen werden können, um den Abstand zum Siebkorb 16 zu verringern. Dieser Vorgang wird basierend auf ein Signal von der Abgleicheinrichtung 14 von der Steuerungsvorrichtung 21 gesteuert.

Die Rütteleinrichtung 20 ist über Säulen 22 am Gehäuse 4 abgestützt, wobei Messelemente (Waage) 24 zur Gewichtsmessung in den Säulen 22 integriert sind, um das auf dem Fließband 18 lastende Gewicht erfassen zu können.

Damit die Abgleicheinrichtung 14 Beurteilungen zur Anzeige bringen kann, ist sie elektronisch mit einer am Tunnelreader 2 vorgesehenen Anzeigevorrichtung 28 verbunden. Diese befindet sich an einer für einen Bediener gut erkennbaren Position. Sie ist vorzugsweise am Tunnelreader 2 angebracht, kann jedoch ebenso Teil einer separaten und in Datenverbindung stehenden Bedieneinrichtung sein. In diesem Fall können die Informationen schnurlos über eine Antennenvorrichtung 30 an die ebenfalls schnurlose Abgleicheinrichtung 14 gesendet werden.

Je nach Einsatzort kann der Tunnelreader 2 auf Beinen oder einer Ablage (nicht dargestellt) abgestellt sein.

Der Tunnelreader 2 kann ebenso als in einer Trennwand montierte Schleuse ausgeführt sein. Solch ein Durchreichesystem mit gegebenenfalls offener Rückwand ist insbesondere im OP-Bereich zur Übergabe des gescannten Sterilguts in den sterilen OP-Bereich vorteilhaft, wo sie als Schnittstelle zwischen dem reinen, sterilen und dem unreinen insterilen Bereich dient.

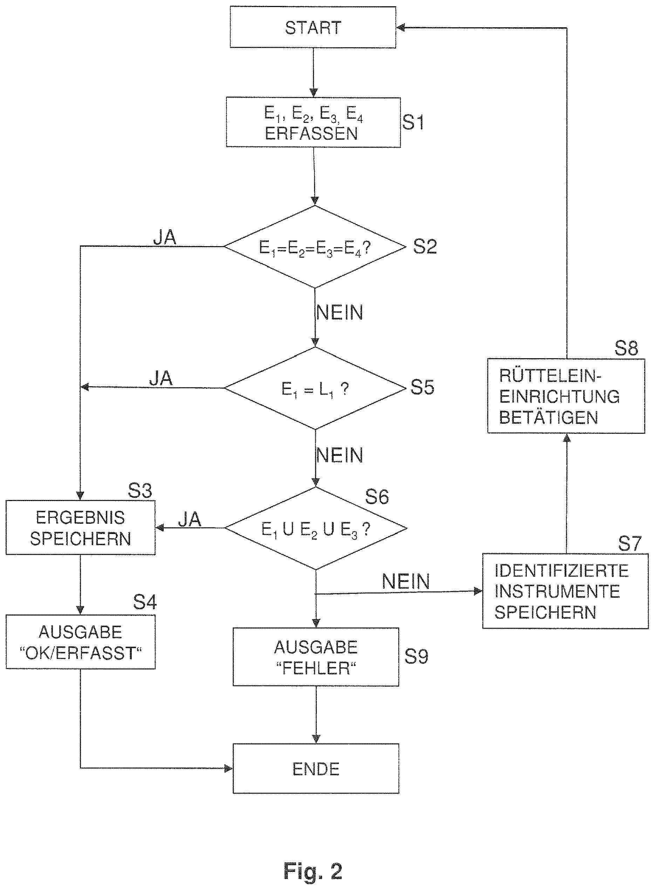

Fig. 2 zeigt beispielhaft ein Ablaufdiagramm zur Erläuterung eines Ablaufs des Tunnelreaders 2 zur gleichzeitigen Identifizierung einer Vielzahl von chirurgischen Instrumenten oder Instrumentgruppen.

Im Folgenden wird ein Erfassungsprozess detailliert unter Verwendung des in Fig. 2 gezeigten Ablaufdiagramms beschrieben, wenn eine Erkennungssteuerung zur gleichzeitigen Identifizierung einer Vielzahl von chirurgischen Instrumenten ausgeführt wird.

Nach dem Start der automatischen Instrumentenerkennung werden in Schritt S1 die im Durchgang 8 des Tunnelreaders 2 befindlichen Instrumente durch die Erfassungseinrichtungen 10, 12, 13, 24 zeitgleich und unmittelbar nacheinander erfasst und die vier Erfassungsergebnisse E₁ bis E₄ ausgelesen. Dabei erfasst die erste Erfassungseinrichtung, d.h. das RFID-Lesegerät 10, das erste Erfassungsergebnis E₁, wobei das jeweilige Instrument durch Übertragung von Informationen über Funk eindeutig identifiziert wird. Das RFID-Lesegerät 10 erweist sich als vergleichsweise zuverlässige Technologie zur Instrumentenerfassung, da es keinen freien Blick auf die Instrumente benötigt und die Instrumente weder frei liegen, noch dem Lesegerät zugewandt sein müssen. Darüber hinaus kann auf dem RFID-Transponder ein Vielzahl an Informationen wie Instrumentenbezeichnung und Teilenummer und Informationen zum Hersteller, zu durchgeführten Wartungen und Reparaturen, Nutzungshäufigkeit, etc. hinterlegt werden, welche mit einer reinen Formerkennung nicht erfasst werden können.

Das RFID-Lesegerät 10 der vorliegenden Erfindung kann in unterschiedlichen Frequenzbereichen arbeiten. Dabei fällt die Wahl je nach Anforderungen an Lesegeschwindigkeit, Reichweite und ähnliches auf den entsprechenden Frequenzbereich. Bevorzugt arbeitet das RFID-Lesegerät 10 im LF-Frequenzbereich (Niedrigfrequenz) bei etwa 125 kHz, im HF-Frequenzbereich (Hochfrequenz) bei etwa 13,56 MHz, im UHF-Frequenzbereich (Ultrahochfrequenz) bei etwa 870 MHz beziehungsweise in der SAW-Technik (Surface Acoustic Wave) beziehungsweise Oberflächenwellen-Technik.

Die zweite Erfassungseinrichtung, d.h. die optische Erkennungseinrichtung beziehungsweise Kamera 12, erfasst Bilder von den Instrumenten, welche durch Abgleichen mit hinterlegten Bildern basierend auf deren Form, Größe und Proportionen einer Instrumentenart und somit dem Erfassungsergebnis E₂ zugewiesen werden. Demnach erkennt die zweite Erfassungseinrichtung mit Hilfe der von der Kamera 12 aufgenommenen Bilder zwar die Art der Instrumente wie beispielsweise eine Schere, Skalpell oder ähnliches und deren Anzahl, kann diese jedoch nicht eindeutig identifizieren. Hierbei ist zu beachten, dass die zu erfassenden Instrumenten möglichst frei liegen sollten, um erkannt zu werden. Das kann vorzugsweise durch die Rüttelvorrichtung 20 erreicht werden. Liegen jedoch so viele Instrumente in einem Siebkorb 16, dass eine im Wesentlichen vollständige Vereinzelung aller zu erfassenden Instrumente nicht möglich ist, können diese dann einzeln auf das Fließband 18 gelegt und von der Kamera 12 erfasst werden.

Als dritte Erfassungseinrichtung erfasst der Barcode-Scanner 13 das Erfassungsergebnis E₃, welches ebenso eine eindeutige Identifizierung und die Erfassung zusätzlicher instrumentenspezifischer Informationen ermöglicht. Hier ist zu beachten, dass der Barcode für den Barcode-Scanner 13 sichtbar sein muss. Das Barcode-Lesegerät kann zum Lesen von eindimensionalen und / oder zweidimensionalen Codes (Data Matrix) geeignet sein.

Die Waage 24 als vierte Erfassungseinrichtung erfasst das Gewicht der zu erfassenden Instrumente als Erfassungsergebnis E₄. Das Gewicht ist am einfachsten zu erfassen, hat vergleichsweise den geringsten Informationsgehalt und dient vorzugsweise als nachrangige Plausibilitätskontrolle zu den anderen Erfassungsergebnissen E₁ bis E₃.

Ein Erfassungsergebnis der Erfassungstechnologien kann eine Anzahl erfasster Instrumente darstellen, damit eine mengenmäßige Vollständigkeit kontrolliert und bestätigt werden kann. Sie kann ebenso Auflistungen der Arten der Instrumente und deren jeweilig Anzahl sein (wie beispielsweise drei Tücher, zwei Klammern, und ähnliches), so dass ein Vorhandensein eines erforderlichen Gegenstands in der richtigen Anzahl kontrolliert werden kann. Das ist insbesondere bei der Kamera 12 der Fall.

Gleichermaßen kann ein Erfassungsergebnis eine Auflistung von exakt identifizierten Instrumenten sein, was das Funk- und Barcode-System ermöglichen.

In Schritt S2 bestimmt die Abgleichvorrichtung 14, ob die Erfassungsergebnisse E₁ bis E₄ übereinstimmen. Wenn die Abgleichvorrichtung 14 in Schritt S2 bestimmt, dass E₁ bis E₄ übereinstimmen ("JA" in Schritt S2), geht die Steuerungsvorrichtung 21 zu Schritt S3. Demnach wurden von den drei Erfassungseinrichtungen 10, 12 und 13 jeweils die gleichen Instrumente erfasst. Ferner stimmt deren erfasstes Gesamtgewicht E₄ mit den hinterlegten Angaben überein. In diesem Fall geht die Steuerungsvorrichtung 21 davon aus, dass alle zu erfassenden Instrumente richtig erfasst wurden und speichert in Schritt S3 das Ergebnis der Erfassung, welches alle identifizierten Instrumente beinhaltet.

Dann ordnet die Steuerungsvorrichtung 21 in Schritt S4 eine Ausgabe "ERGEBNIS IN ORDNUNG UND ERFASST" an der Anzeigeeinrichtung 28 an und der Ablauf wird beendet. Statt einer Textanzeige kann auch ein bestimmtes Symbol oder lediglich ein grünes Licht angezeigt werden

Wird jedoch in Schritt S2 eine negative Beurteilung bestimmt ("NEIN" in Schritt S2), geht die Steuerungsvorrichtung 21 zu Schritt S5. Hier wird bestimmt, ob das Erfassungsergebnis E₁ mit einem vorbestimmten, in der Abgleicheinrichtung 14 hinterlegten Datensatz bzw. Instrumentenliste L₁ übereinstimmt. Wird in Schritt S5 bestimmt, dass E₁ mit L₁ übereinstimmt ("JA" in Schritt S5), geht die Steuerungsvorrichtung 21, wie oben beschrieben, zu den Schritten S3 und S4 und der Ablauf wird beendet. In diesem Fall wird das aussagekräftigste und zuverlässigste E₁ von der Steuerungsvorrichtung 21 gegenüber den anderen Technologien priorisiert.

Wird jedoch in Schritt S5 eine negative Beurteilung bestimmt ("NEIN" in Schritt S5), geht die Steuerungsvorrichtung 21 weiter zu Schritt S6.

In Schritt S6 bestimmt die Abgleichvorrichtung 14, ob E₁ in Kombination mit den anderen Ergebnissen E₂ und E₃ den Inhalt der Liste L₁ enthält. Wenn einige wenige Instrumente der Liste L₁ nicht durch das RFID-Lesegerät 10 erfasst wurden, wird auf die beiden anderen Ergebnisse zurück gegriffen, um dennoch ein positives Ergebnis zu erzielen.

Wird in Schritt S6 eine positive Beurteilung bestimmt ("JA" in Schritt S6), geht die Steuerungsvorrichtung 21, wie oben beschrieben, weiter zu den Schritten S3 und S4 und der Ablauf wird beendet.

Wird jedoch in Schritt S6 eine negative Beurteilung bestimmt ("NEIN" in Schritt S6), geht die Steuerungsvorrichtung 21 zu Schritt S7, in welchem die aktuell identifizierten Instrumente als ein Zwischenergebnis gespeichert werden. Im folgenden Schritt 8 wird die Rütteleinrichtung 20 durch die Steuerungsvorrichtung 21 für eine vorbestimmte Zeitdauer betätigt, um die aktuelle Ordnung der im Siebkorb 16 befindlichen Instrumente zu verändern. Dadurch sollen insbesondere verdeckte und somit für die Kamera 12 und den Barcode-Scanner 13 nicht sichtbare Instrumente freigelegt werden.

Dann geht die Steuerungsvorrichtung 21 weiter zu Schritt S1 und es schließt sich der bereits oben beschriebene Ablauf, nachfolgend dem Schritt S1, an. Allerdings ist davon auszugehen, dass die beim ersten Lesevorgang des RFID-Lesegeräts nicht erfassten Instrumente unter Umständen aufgrund einer Fehlfunktion der RFID-Tags auch weiterhin nicht erfasst werden können, so dass auf die redundanten Systeme zurückgegriffen wird. Liegen nach Schritt S2 vier übereinstimmende Erfassungsergebnisse E₁ bis E₄ oder andernfalls nach Schritt S5 eine Überstimmung von dem erneut erfassten E₁ mit L₁ vor, geht der Ablauf, wie bereits oben beschrieben, zu den Schritten S3 und S4 und der Ablauf wird beendet.

Ist die Bestimmung in beiden Schritten S2 und S5 negativ, geht der Ablauf zu Schritt S6. In diesem Schritt bestimmt die Abgleichvorrichtung 14, wie oben beschrieben, ob eine Kombination der Erfassungsergebnisse E₁ bis E₃ den Inhalt der Liste L₁ enthält. Sollte dies wieder nicht der Fall sein, vergleicht die Abgleichvorrichtung 14 in einer Zusammenschau aller Erfassungsergebnisse, ob alle auf der Liste L₁ aufgeführten Instrumente identifiziert werden konnten. Da mittels der Funktechnologie und dem Barcode-Scanner die Instrumente eindeutig identifiziert werden, wird selbst beim Vergleichen der Erfassungsergebnisse von mehreren Lesevorgängen dasselbe Instrument nicht doppelt gezählt.

Je nach Anzahl der Durchläufe der Schritte S7 und S8 liegen unterschiedlich viele Erfassungsergebnisse vor. Wie oft ein erneuter Versuch einer vollständigen Erfassung durchlaufen wird, kann im Vorfeld gespeichert sein oder von dem Bediener gesteuert werden.

Abweichend von dem oben gezeigten Ausführungsbeispiel kann der Tunnelreader 2 sowohl in seiner Länge als auch in seiner Höhe variieren, so dass Siebkörbe 16 unterschiedlicher Größen und eine Vielzahl von Siebkörben 16 auf einmal von dem Tunnelreader 2 aufgenommen werden können, solange sichergestellt ist, dass die erfassten Instrumente einem bestimmten Siebkorb 16 zugeordnet werden können.

An der Anzeigeeinrichtung 28 können sowohl die Beurteilungen der Abgleicheinrichtung 14 als auch weitere Informationen, wie beispielsweise Hinweise auf erforderliche Sonderreinigungsprogramme, Hinweise auf erforderliche Vorbereitungsmaßnahmen (Demontage, Vorbehandlung), Inspektionsanweisungen sowie Anweisungen zum Austausch/Entsorgen einzelner Instrumente, zur Anzeige gebracht werden. Diese Informationen können beispielsweise über eine an dem Tunnelreader 2 angebrachten Antenne 30 an die Anzeigevorrichtung 28 mit einer Empfängereinheit (nicht dargestellt) gesendet werden.

Die Waage 24 zur Gewichtsmessung der Erfassungsgegenstände kann ebenso an der Gehäuseunterseite, in den Beinen 30 oder ähnliches angeordnet sein.

Die Rüttelvorrichtung 20 kann ebenso an einem Bereich vor oder hinter dem Gehäuse 4 angeordnet sein, so dass die zu erfassenden Instrumente dementsprechend vor oder hinter dem Tunnelreader 2 geschüttelt werden. Indem die Rüttelvorrichtung 20 außerhalb des Gehäuses angeordnet wird, kann die Bedienperson den Rüttelprozess beobachten und steuern.

Anstelle einer durch einen Elektromotor angetriebenen Teleskopvorrichtung 23 können die Erfassungseinrichtungen ebenso über einen Seilzug in Richtung des Siebkorbs 16 hinabgelassen und gegebenenfalls in ihrer Orientierung verändert werden. Ebenso können die Erfassungseinrichtungen jeweils an einer senkrecht zur Längsrichtung des Tunnelreaders 2 verlaufenden Schiene angeordnet sein, wobei die Schiene von ihrer Mitte in Längsrichtung in Richtung der beiden Enden nach unten gebogen ist.

Ferner können die Erfassungseinrichtungen jeweils eine separate Leseeinheit aufweisen, so dass nur diese beweglich ist und eine Lesedistanz zu den Instrumenten verändern kann.

Offenbart sind demnach eine Einrichtung sowie ein Verfahren, die für eine multiple Auslesbarkeit von kompletten Instrumentsiebkörben geeignet sind. Dies wird insbesondere durch eine Kombination verschiedener Erkennungssysteme in einem Gerät, welche untereinander vernetzt sind und miteinander kommunizieren, erreicht. Dabei werden die jeweiligen Erfassungsergebnisse untereinander sowie mit vorbestimmten Instrumentenlisten verglichen. Das Gerät erfasst also über ein Funkerkennungssystem und ein zusätzliches optisches Erkennungssystem und / oder einen Barcode-Scanner Instrumenteninformationen, wobei eine Waage durch eine Plausibilitätskontrolle die Erfassungsergebnisse kontrolliert. Unterstützend kommen ein Rüttelsystem und / oder ein Fließbandsystem zum Einsatz, welche auf unterschiedlichste Weisen etwa durch eine kontinuierliche und / oder intermittierende Weise betätigt werden können. Somit werden Kombigeräte mit mehreren integrierten Instrumentenerfassungssystemen mit dem Ziel geschaffen, die Erkennungswahrscheinlichkeit von Siebkorbinhalten zu maximieren. Infolge einer annähernd hundertprozentigen Erfassungssicherheit werden durch Automatisieren des Lesevorgangs Zeit und Kosten gespart und die Sicherheit im Umgang mit chirurgischen Instrumenten nimmt zu.

Die Einrichtung ist trotz einer Metallumgebung auf Grund von Instrumenten, des Siebs und von Containern dazu geeignet, ungeordnete und einander teilweise oder vollständig überlappende Instrumente im Siebkorb, unabhängig von der Anzahl und der Lage der jeweiligen Instrumente, vollständig zu erfassen.

### Bezugszeichenliste

- 2: Tunnelreader
- 4: Gehäuse
- 6: Öffnung
- 8: Durchgang
- 10: erste Erfassungseinrichtung bzw. RFID-Lesegerät
- 12: zweite Erfassungseinrichtung bzw. optisches Erkennungseinrichtung (Kamera)
- 13: Barcode-Scanner
- 14: Abgleicheinrichtung
- 16: Siebkorb
- 18: Platte beziehungsweise Fließband
- 20: Rütteleinrichtung
- 21: Steuerungsvorrichtung
- 22: Haltevorrichtungen bzw. Säulen
- 23: Teleskophaltevorrichtung mit Elektromotor (nicht dargestellt)
- 24: Waage
- 28: Anzeigeeinrichtung bzw. Display
- 30: Antenne

## Patentansprüche

1. Einrichtung zum gleichzeitigen Identifizieren von einer Vielzahl, vorzugsweise unterschiedlicher, chirurgischer Instrumente beziehungsweise Instrumentgruppen, mit:
einer ersten Erfassungseinrichtung (10) mit einer ersten Erfassungstechnologie, insbesondere einer RFID-Technologie, zum Erfassen von Instrumenten und gegebenenfalls zugehörigen instrumentenspezifischen Informationen (E₁);
**gekennzeichnet durch**
eine zweite Erfassungseinrichtung (12) mit einer zweiten, zur ersten unterschiedlichen Erfassungstechnologie mit einer optischen Instrumentenformerkennung zum Erfassen von Instrumenten und gegebenenfalls zugehörigen instrumentenspezifischen Informationen (E₂); und
eine Abgleicheinrichtung (14) zum Abgleichen der Erfassungsergebnisse (E₁, E₂) der beiden Erfassungseinrichtungen (10, 12), wobei
die Abgleicheinrichtung (14) hinsichtlich der Richtigkeit der erfassten Instrumente eine positive Beurteilung ausgibt, wenn die beiden Erfassungsergebnisse (E₁, E₂) übereinstimmen.

2. Einrichtung nach Anspruch 1, **dadurch gekennzeichnet, dass**
die Abgleicheinrichtung (14) die beiden Erfassungsergebnisse (E₁, E₂) mit einem vorbestimmten Datensatz (L₁), insbesondere einer Instrumentenliste oder einer Siebkorbliste, vergleicht und eine positive Beurteilung ausgibt, wenn zumindest eines der beiden Erfassungsergebnisse (E₁, E₂) oder zumindest eine Kombination der beiden Erfassungsergebnisse (E₁, E₂) mit dem vorbestimmten Datensatz (L₁) übereinstimmt.

3. Einrichtung nach Anspruch 1 oder 2, ferner **gekennzeichnet durch**:
eine Rütteleinrichtung (20) und / oder einer Fließbandvorrichtung (18) zum Vereinzeln der Instrumente, welche zeitlich und / oder örtlich vor- oder nachgeschaltet ist; und
eine Steuerungsvorrichtung (21) zum Steuern der Rüttel- und / oder Fließbandeinrichtung (20, 18), die zumindest einmal die Rüttel- und / oder Fließbandeinrichtung (20, 18) für eine vorbestimmte Zeitdauer betätigt, bevor die Instrumenteninformationen erfasst oder wieder erfasst werden.

4. Einrichtung nach Anspruch 2 oder 3, ferner **gekennzeichnet durch**:
eine Anzeigevorrichtung (28), mit der eine Beurteilung und produktspezifische Informationen zur Anzeige gebracht werden, wobei
der Datensatz (L₁) Zusatzinformationen wie beispielsweise eine spezielle Behandlung und / oder Handhabung hinsichtlich der Instrumente enthält, die von der Anzeigevorrichtung (28) ausgegeben werden, wenn das entsprechende Instrument identifiziert wird.

5. Einrichtung nach einem der Ansprüche 1 bis 4, ferner **gekennzeichnet durch**:
Mittel (23) zum Ändern einer relativen Orientierung und / oder eines relativen Abstands zwischen den Instrumenten und den Erfassungseinrichtungen (10, 12).

6. Einrichtung nach einem der Ansprüche 1 bis 5, **dadurch gekennzeichnet, dass**:
die Einrichtung als ein Tunnelreader (2) ausgebildet ist; und
der Tunnelreader (2) als abgekapseltes Gehäuse (4) ausgebildet ist, welches in Form einer Durchreiche ausgebildet ist.

7. Verfahren zum gleichzeitigen Identifizieren von einer Vielzahl an, vorzugsweise unterschiedlicher, chirurgischer Instrumente beziehungsweise Instrumentgruppen, mit dem Schritt:
Erfassen der Instrumente mit einer ersten Erfassungseinrichtung (10) mittels einer ersten Erfassungstechnologie, insbesondere einer RFID-Technologie; **gekennzeichnet durch** die Schritte:
Erfassen der Instrumente mit einer zweiten Erfassungseinrichtung (12) mittels einer zweiten Erfassungstechnologie mit einer optischen Instrumentenformerkennung; und
Vergleichen der beiden Erfassungsergebnisse (E₁, E₂), wobei eine positive Beurteilung ausgegeben wird, wenn die beiden Erfassungsergebnisse (E₁, E₂) übereinstimmen;

8. Verfahren nach Anspruch 7, ferner **gekennzeichnet durch** den Schritt:
Vergleichen der beiden Erfassungsergebnisse (E₁, E₂) mit einer Instrumentenliste (L₁), wobei eine positive Beurteilung ausgegeben wird, wenn zumindest eines der Erfassungsergebnisse (E₁, E₂) oder zumindest eine Kombination aus beiden Erfassungsergebnissen (E₁, E₂) mit der Instrumentenliste (L₁) übereinstimmt

9. Verfahren nach Anspruch 7 oder 8, ferner **gekennzeichnet durch** den Schritt:
Betätigen einer Rüttelvorrichtung (20) für eine vorbestimmte Zeitdauer, wenn keine positive Beurteilung ausgegeben werden kann, wobei nach Betätigen der Rütteleinrichtung (20) die Erfassungsergebnisse (E₁, E₂) erneut erfasst werden.

## Claims

1. A device for simultaneously identifying a plurality of preferably different surgical instruments or instrument groups, comprising:
a first detecting device (10) including a first detection technology, especially an RFID technology, for detecting instruments and possibly related instrument-specific information (E₁);
**characterized by**
a second detecting device (12) including a second detection technology different from the first detection technology, comprising an optical instrument shape identification, for detecting instruments and possibly related instrument-specific information (E₂); and
a comparing device (14) for comparing the detection results (E₁, E₂) of the two detection devices (10, 12), wherein
the comparing device (14) outputs a positive evaluation regarding the correctness of the detected instruments if the two detection results (E₁, E₂) are matching.

2. The device according to claim 1, **characterized in that**
the comparing device (14) compares the two detection results (E₁, E₂) to a predetermined data set (L₁), especially an instrument list or a basket list, and outputs a positive evaluation if at least one of the two detection results (E₁, E₂) or at least a combination of the two detection results (E₁, E₂) matches the predetermined data set (L₁).

3. The device according to claim 1 or 2, further **characterized by**:
a vibrating means (20) and/or a conveyor belt device (18) for separating the instruments arranged upstream or downstream in time and/or in place; and
a control device (21) for controlling the vibrating and/or conveyor belt means (20, 18) which at least once actuates the vibrating and/or conveyor belt means (20, 18) for a predetermined period of time before the instrument information is detected or detected again.

4. The device according to claim 2 or 3, further **characterized by**:
a display device (28) by which an evaluation and product-specific information are displayed, wherein
the data set (L₁) contains additional information, such as a specific treatment and/or handling as regards the instruments, output by the display device (28) when the respective instrument is identified.

5. The device according to any one of the claims 1 to 4, further **characterized by**:
means (23) for changing a relative orientation and/or a relative distance between the instruments and the detecting devices (10, 12).

6. The device according to any one of the claims 1 to 5, **characterized in that**:
the device is configured as tunnel reader (2); and
the tunnel reader (2) is an encapsulated housing (4) in the form of a pass-through.

7. A method for simultaneously identifying a plurality of preferably different surgical instruments or instrument groups, comprising the step of:
detecting the instruments by a first detecting device (10) by means of a first detection technology, especially an RFID technology; **characterized by** the steps of:
detecting the instruments by a second detecting device (12) by means of a second detecting technology including optical instrument shape identification; and
comparing the two detection results (E₁, E₂), wherein a positive evaluation is output if the two detection results (E₁, E₂) are matching.

8. The method according to claim 7, further **characterized by** the step of:
comparing the two detection results (E₁, E₂) to an instrument list (L₁), wherein a positive evaluation is output if at least one of the detection results (E₁, E₂) or at least a combination of both detection results (E₁, E₂) matches the instrument list (L₁).

9. The method according to claim 7 or 8, further **characterized by** the step of:
actuating a vibrating means (20) for a predetermined period of time when no positive evaluation can be output, wherein after actuating the vibrating means (20) the detection results (E₁, E₂) are detected again.

## Revendications

1. Système servant à identifier de manière simultanée une pluralité d'instruments ou de groupes d'instruments chirurgicaux de préférence différents, comprenant :
un premier système de détection (10) avec une première technologie de détection, en particulier une technologie FRID, servant à détecter des instruments et, le cas échéant, des informations (E₁) associées spécifiques aux instruments ;
**caractérisé par**
un deuxième système de détection (12) avec une deuxième technologie de détection différente de la première, avec une identification optique de formes d'instruments servant à détecter des instruments et, le cas échéant, des informations (E₂) associées spécifiques aux instruments ; et
un système d'alignement (14) servant à aligner les résultats de détection (E₁, E₂) des deux systèmes de détection (10, 12), dans lequel
le système d'alignement (14) délivre un avis positif eu égard à l'exactitude des instruments détectés quand les deux résultats de détection (E₁, E₂) coïncident.

2. Système selon la revendication 1, **caractérisé en ce que**
le système d'alignement (14) compare les deux résultats de détection (E₁, E₂) à un jeu de données (L₁) prédéfinis, en particulier à une liste d'instruments ou à une liste de panier de criblage, et délivre un avis positif quand au moins un des deux résultats de détection (E₁, E₂) ou au moins une combinaison des deux résultats de détection (E₁, E₂) coïncide avec le jeu de données (L₁) prédéfini.

3. Système selon la revendication 1 ou 2, **caractérisé en outre par** :
un système agitateur (20) et/ou un dispositif à chaîne de montage (18) servant à trier les instruments, lequel est monté dans le temps antérieurement ou ultérieurement et/ou spatialement en amont ou en aval ; et
un dispositif de commande (21) servant à commander le système agitateur et/ou le dispositif à chaîne de montage (20, 18), qui actionne du moins une fois le système agitateur et/ou le dispositif à chaîne de montage (20, 18) pour une période de temps prédéfinie avant que les informations des instruments ne soient détectées ou ne soient à nouveau détectées.

4. Système selon la revendication 2 ou 3, **caractérisé en outre par** :
un dispositif d'affichage (28), lequel permet d'afficher un avis et des informations spécifiques aux instruments, dans lequel
le jeu de données (L₁) contient des informations supplémentaires telles que par exemple un traitement et/ou une manipulation spécifique eu égard aux instruments, lesquelles sont délivrées par le dispositif d'affichage (28) lorsque l'instrument correspondant est identifié.

5. Système selon l'une quelconque des revendications 1 à 4, **caractérisé en outre par** :
des moyens (23) servant à modifier une orientation relative et/ou une distance relative entre les instruments et les systèmes de détection (10, 12).

6. Système selon l'une quelconque des revendications 1 à 5, **caractérisé en ce que** :
le système est réalisé sous la forme d'un lecteur tunnel (2) ; et
le lecteur tunnel (2) est réalisé sous la forme d'un boîtier (4) isolé, qui est réalisé sous la forme d'un portique de passage.

7. Procédé servant à identifier de manière simultanée une pluralité d'instruments ou de groupes d'instruments chirurgicaux de préférence différents, comprenant l'étape suivante consistant à :
détecter les instruments avec un premier système de détection (10) au moyen d'une première technologie de détection, en particulier une technologie RFID ;
**caractérisé par** les étapes suivantes consistant à :
détecter les instruments avec un deuxième système de détection (12) au moyen d'une deuxième technologie de détection comprenant une identification optique de formes d'instruments ; et
comparer les deux résultats de détection (E₁, E₂), dans lequel un avis positif est délivré quand les deux résultats de détection (E₁, E₂) coïncident.

8. Procédé selon la revendication 7, **caractérisé en outre par** l'étape suivante consistant à :
comparer les deux résultats de détection (E₁, E₂) à une liste d'instruments (L₁), dans lequel un avis positif est délivré quand au moins un des résultats de détection (E₁, E₂) ou au moins une combinaison des deux résultats de détection (E₁, E₂) coïncide avec la liste d'instruments (L₁).

9. Procédé selon la revendication 7 ou 8, **caractérisé en outre par** l'étape suivante consistant à :
actionner un système agitateur (20) pour une période de temps prédéfinie, quand aucun avis positif ne peut être délivré, dans lequel après l'actionnement du système agitateur (20), les résultats de détection (E₁, E₂) sont détectés à nouveau.
